Europäisches Patentamt

European Patent Office

Office européen des brevets

(11) Veröffentlichungsnummer: **0 369 141**
**A1**

## (12) EUROPÄISCHE PATENTANMELDUNG

(21) Anmeldenummer: **89117881.6**

(51) Int. Cl.5: **A61K 37/64, A61K 37/02**

(22) Anmeldetag: **27.09.89**

<table>
<tr><td>

Die Bezeichnung der Erfindung wurde geändert (Richtlinien für die Prüfung im EPA, A-III, 7.3).

(30) Priorität: **05.10.88 DE 3833764**

(43) Veröffentlichungstag der Anmeldung:
**23.05.90 Patentblatt 90/21**

(84) Benannte Vertragsstaaten:
**AT BE CH DE ES FR GB IT LI NL SE**

</td><td>

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Raddatz, Peter, Dr.**
**Grafenstrasse 37**
**D-6100 Darmstadt(DE)**
Erfinder: **Schmitges, Claus J., Dr.**
**Karolinger Strasse 5**
**D-6114 Gross Umstadt(DE)**

</td></tr>
</table>

(54) **Aspartylproteasen-Inhibitoren zur Behandlung von durch Retroviren verursachten Erkrankungen.**

(57) Die Erfindung betrifft die Verwendung von Aminosäurederivaten, die die Aktivität von Retroviren-Proteasen hemmen und das Strukturelement der Formel I
$-NR^1-CHR^2-CR^3-(CHR^4)_n-CO-$
aufweisen, worin
$R^1$, $R^4$, $R^5$, $R^6$ jeweils H oder Alkyl,
$R^2$ H, Alkyl, jeweils substituiertes oder unsubstituiertes Aryl, Arylalkyl, Cycloalkyl, Cycloalkylalkyl, Het oder Het-alkyl,
$R^3$ (H, OH), (H, $NR^5R^6$) oder O,
Het einen 5- oder 6-gliedrigen N-, S- oder O-haltigen gesättigten oder ungesättigten Heterocyclus und
n 1 oder 2
bedeuten, oder von deren physiologisch unbedenklichen Salzen für die Behandlung von Erkrankungen, die durch Retroviren verursacht werden.

EP 0 369 141 A1

## Arzneimittel zur Behandlung von durch Retroviren verursachte Erkrankungen

Die Erfindung betrifft ein Arzneimittel für die Behandlung von durch Retroviren verursachte Erkrankungen mit Aminosäurederivaten, die die Aktivität von Retroviren-Proteasen zu hemmen vermögen oder mit den pharmakologisch annehmbaren Salzen dieser Verbindungen.

Retroviren sind für eine Reihe von ernsthaften und oft letalen Erkrankungen bei Tier und Mensch verantwortlich. Unter den Retroviren sind insbesondere zu nennen HTLV-III/LAV (HIV-1), ferner HTLV-I und HTLV-II, Rous-Sarkom-Virus, Friend-Virus und Rauscher-Leukämie-Virus, unter den Erkrankungen dementsprechend in erster Linie AIDS (acquired immunodeficiency syndrom) und dessen Vorstufen.

Die Behandlung von entsprechenden Erkrankungen kann nur dann erfolgreich und von Dauer sein, wenn der Virus bzw. seine Vermehrung in der Wirtszelle direkt und spezifisch bekämpft wird. Geeignete Arzneimittel sind noch wenig bekannt. So kann beispielsweise die Replikation des HTLV-III/LAV-Virus durch D-Penicillamin in vitro und in vivo gehemmt werden (vgl. Chandra u. Sarin, Arzneimittelforschung (Drug. Res.) 36 (I) (1986) 184-186; Schulof et al., ibid. 36 (II) (1986) 1531-34). Das Mittel, das unter dem Namen Retrovir im Handel ist, ist aber nur begrenzt einsetzbar, da es oft erhebliche Nebenwirkungen, wie Anämie, Leukopenie, Allergien usw. auslöst.

Der Erfindung lag somit die Aufgabe zugrunde, ein Arzneimittel zur Verfügung zu stellen, das erfolgreich auch ohne ernstere Nebenwirkungen zur Bekämpfung von Retroviren bzw. zur Behandlung von durch sie verursachten Erkrankungen eingesetzt werden kann.

Überraschend wurde nun gefunden, daß Aminosäurederivate, die als Aspartylproteasen-Inhibitoren wirksam sind und das Strukturelement der Formel I

$-NR^1-CHR^2-CR^3-(CHR^4)_n-CO-$

aufweisen, worin

$R^1$, $R^4$, $R^5$, $R^6$ jeweils H oder Alkyl,

$R^2$ H, Alkyl, jeweils substituiertes oder unsubstituiertes Aryl, Arylalkyl, Cycloalkyl, Cycloalkylalkyl, Het oder Het-alkyl,

$R^3$ (H, OH), (H, $NR^5R^6$) oder O,

Het einen 5- oder 6-gliedrigen N-, S- oder O-haltigen gesättigten oder ungesättigten Heterocyclus und

n 1 oder 2

bedeuten, oder deren physiologisch unbedenkliche Salze auch effektiv die Aktivität einer virus-kodierten und somit virus-spezifischen, für die Retroviren-Vermehrung essentiellen Protease hemmen.

Aminosäureverbindungen, die das Strukturelement der Formel I enthalten und zugleich als Aspartylproteasen-Inhibitoren fungieren können, sind beispielsweise aus den folgend genannten offengelegten Schutzrechten bekannt:

EP 0 155 809, EP 0 156 319, EP 0 161 588, EP 0 179 352, EP 0 184 855, EP 0 188 271, EP 0 212 903, EP 0 220 665, EP 0 222 283, EP 0 229 667, EP 0 244 083, EP 0 249 096, EP 0 262 318, EP 0 264 795 und EP 0 272 444.

Gegenstand der Erfindung ist so die Verwendung von Aminosäurederivaten, die die Aktivität von Retroviren-Proteasen hemmen und das Strukturelement der Formel I aufweisen, oder von deren physiologisch unbedenklichen Salzen zur Herstellung eines Arzneimittels für die Behandlung von durch Retroviren verursachten Erkrankungen.

Gegenstand der Erfindung ist insbesondere die Verwendung solcher Verbindungen zur Herstellung eines Arzneimittels für die Behandlung von AIDS.

Als pharmazeutisch unbedenkliche Salze der Verbindungen mit dem Strukturelement der Formel I eignen sich solche, die von Mineralsäuren oder organischen Säuren ableitbar sind, beispielsweise von Schwefelsäure, Salpetersäure, Halogenwasserstoffsäuren wie Chlorwasserstoffsäure oder Bromwasserstoffsäure, ferner organischen Säuren, insbesondere aliphatischen, alicyclischen, araliphatischen, aromatischen oder heterocyclischen ein- oder mehrbasigen Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotin- oder Isonicotinsäure, Methan- oder Ethansulfonsäure, 2-Hydroxyethansulfonsäure, Benzolsulfonsäure, p-Toluolsulfonsäure, Naphthalin-mono- und disulfonsäuren oder Laurylschwefelsäure.

Als Verbindungen, die für die erfindungsgemäße Verwendung eingesetzt werden können, eignen sich insbesondere beispielsweise alle, die von den oben aufgeführten Schutzrechten umfaßt werden. Besonders bevorzugt zur Bekämpfung von Retroviren und den durch sie verursachten Erkrankungen, insbesondere von AIDS, sind jene Verbindungen aus der EP-A-0 161 588, EP-A-0 179 352, EP-A-0 249 096, EP-A-0 262 318, EP-A-0 264 795, EP-A-0 272 444 sowie desweiteren aus der DE 3 707 879, DE 3 717 631 und DE 3 817 449. Hiervon sind wiederum solche Verbindungen in besonderem Maße geeignet, die in der EP-A-0 161 588, EP-A-0 249 096 und der DE 3 817 449 genannt sind.

Herstellung und Eigenschaften der Verbindungen, die für die erfindungsgemäße Verwendung eingesetzt werden, sind in den zitierten Schutzrechten ausführlich erläutert.

Die Hemmung der Aktivität der Retroviren-Protease kann beispielsweise nach einer Methode von Katoh et al., Nature 329, 654-656 (1987) oder Von der Helm et al., Proc. Natl.Acad. Sci. USA Vol. 85 p 6612-16 (1988) nachgewiesen bzw. getestet werden.

Die Aminosäurederivate mit dem Strukturelement der Formel I oder deren Salze können oral, parenteral, durch Sprühinhalation oder rektal in geeigneten Formulierungen angewendet werden, die übliche, nicht-toxische, pharmakologisch annehmbare Träger, Adjuvantien und Verdünnungsmittel enthalten. Der hier verwendete Ausdruck "parenteral" schließt subkutane Injektionen sowie auch intravenöse, intramuskuläre, intrasternale Injektionen und Infusionen ein. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations- Sprays eignen und mit den Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Entsprechende orale Formulierungen können auch beispielsweise Süßungsmittel, wie Glycerin, Sorbit oder Sucrose oder auch Milderungsmittel, Schutzmittel, Geschmacksverbesserer und/oder Farbstoffe enthalten. Zur rektalen Anwendung dienen Suppositorien, zur parenteralen Applikation Lösungen, vorzugweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die Verbindungen können auch lyophilisiert und die erhaltenen Lyophilisate z.B. zur Herstellung von Injektionspräparaten verwendet werden. Die angegebenen Zubereitungen können gegebenenfalls Suspensionsmittel, wie Natriumcarboxymethylcellulose, Methylcellulose, Hydroxypropylmethylcellulose, Natriumalginat, Polyvinylpyrrolidon, Tragant-Gummi und Gummi arabicum und/oder Dispergier- bzw. Befeuchtungsmittel, wie Lecithin, enthalten. Die Zubereitungen können auch sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine oder biologische Immunmodulatoren wie Interferon oder Interleukine.

Die Verbindungen die gemäß der neuen Verwendung eingesetzt werden, werden in der Regel in Analogie zu anderen bekannten, im Handel befindlichen Aminosäurederivaten bzw. Peptiden verabreicht, vorzugsweise in Dosierungen zwischen etwa 10 mg und 10 g, insbesondere zwischen 50 mg und 5 g pro Dosierungseinheit. Die tägliche Dosierung liegt vorzugsweise zwischen 0,1 bis 500 mg, insbesondere zwischen 0,5 bis 100 mg/kg Körpergewicht bei enteraler Applikation und zwischen 0,1 bis 100 mg, insbesondere zwischen 0,3 bis 30 mg/kg Körpergewicht bei parenteraler Applikation. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Die Arzneimittel können, falls erwünscht, auch einen oder mehrere weitere Wirkstoffe enthalten, z.B. biologische Immunmodulatoren wie Interferon oder Interleukine.

Die in den folgenden Beispielen aufgeführten Abkürzungen von Aminosäureresten stehen für die Reste -NR'-R"-CO-, in der Regel -NH-CHR-CO- (worin R, R' und R" die für jede Aminosäure bekannte spezifische Bedeutung haben) folgender Aminosäuren:

Abu 2-Aminobuttersäure
Ada 3-(1-Adamantyl)-alanin
AHCP 4S-Amino-3S-hydroxy-5-cyclohexylpentansäure
AHPP 4S-Amino-3S-hydroxy-5-phenylpentansäure
AHMO 5S-Amino-4S-hydroxy-7-methyloctansäure

AHPH 5S-Amino-4S-hydroxy-6-phenylhexansäure
AHCH 5S-Amino-4S-Hydroxy-6-cyclohexylhexansäure
AHIM 5S-Amino-4S-hydroxy-2-isopropyl-7-methyloctansäure
AHIP 5S-Amino-4S-hydroxy-2-isopropyl-6-phenylhexansäure
AHIC 5S-Amino-4S-hydroxy-2-isopropyl-6-cyclohexylhexansäure
AHDM 5S-Amino-4S-hydroxy-2,7-dimethyloctansäure
AHMP 5S-Amino-4S-hydroxy-2-methyl-6-phenylhexansäure
AHMC-5S-Amino-4S-hydroxy-2-methyl-6-cyclohexylhexansäure
Ala Alanin
βAla beta-Alanin
Arg Arginin
Cal 3-Cyclohexylalanin
DACP 3S,4S-Diamino-5-Cyclohexylpentansäure
DAMH 3S,4S-Diamino-6-methylheptansäure
DAPP 3S,4S-Diamino-5-phenylpentansäure
Gly Glycin
Gln Glutamin
His Histidin
Ile Isoleucin
Leu Leucin
Mal 3-(p-Methoxyphenyl)-alanin
Met Methionin
Nle Norleucin
Nva Norvalin
Phe Phenylalanin
Ser Serin
Sta Statin
Thr Threonin
Trp Tryptophan
Val Valin
    Ferner bedeutet nachstehend
ADPA N-2-Amino-5,6-dimethyl-3-pyrazinylmethyl-amid
AMPA N-4-Amino-2-methyl-5-pyrimidinylmethyl-amid
BOC tert-Butoxycarbonyl
CBZ Benzyloxycarbonyl
ETOC Ethoxycarbonyl
IPOC Isopropoxycarbonyl
OMe Methylester
OEt Ethylester

Beispiele:

1. Injektionsgläser

Eine Lösung von 100 g BOC-Pro-Phe-His-DAMH-Ile-Phe-OMe und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.
Analog werden Injektionsgläser mit folgenden Wirkstoffen hergestellt:

| | Fp °C |
|---|---|
| BOC-Phe-His-DAMH-Ile-Sta-OMe | 140-2 |
| BOC-Phe-Abu-DAMH-Ile-N-(2-phenylethylamid) | 195 |
| BOC-Phe-Asn-DAMH-Ile-Phe-OMe | 197-8 |
| BOC-Phe-Abu-DAMH-Ile-Val-OEt | |
| BOC-Phe-Abu-DAMH-Abu-Phe-OH | |
| BOC-Abu-DAMH-Ile-Phe-NH$_2$ | 186 |
| BOC-Asn-DAMH-Ile-Phe-OMe | 180 |
| BOC-Phe-ßAla-DAPP-Ile-AMPA | 225-7 |
| BOC-Phe-Ala -DAPP-Ile-AMPA | 163-5 |
| BOC-Phe-Val -DAPP-Ile-AMPA | 232-4 |
| BOC-Leu-ßAla-DAPP-Ile-AMPA | 226-7 |
| BOC-Gln-Asn -DAPP-Ile-Val-OMe | 192-3 |
| BOC-Phe-Asn -DAPP-Gln-Val-OMe | 165-7 |
| BOC-Leu-Asn -DAPP-Ile-Ser-OMe | 185-7 |
| BOC-Leu-Asn -DAPP-Ile-Gly-OMe | 186-8 |
| BOC-Leu-Abu -DAPP-Ile-Val-OMe | 175-6 |
| BOC-Leu-Ala -DAPP-Ile-Val-OMe | 179-80 |
| CBZ-Phe-Abu -DAPP-Abu-Phe-OH | |
| IPOC-Phe-ßAla-DACP-Ile-AMPA | |
| BOC-Abu -Sta-Ile-DAMH-OMe | 126-7 |
| BOC-Phe-Abu -Sta-Ile-DAMH-OMe | 190 |
| BOC-Asn -Sta-Ile-DAMH-OMe | 178-9 |
| BOC-Phe-Asn -Sta-Ile-DAMH-OMe | 181 |
| BOC-Leu-Abu -Sta-Ile-DAMH-OMe | 197 |
| BOC-Gln-Abu -Sta-Ile-DAMH-OMe | 182-3 |
| CBZ-Phe-Asn -Sta-Ile-Val-OMe | |
| CBZ-Leu-Asn -Sta-Ile-Ser-OMe | |
| BOC-Phe-ßAla-Sta-Ile-AMPA | |

CBZ-His-DAPP-Ile-Phe-OMe

BOC-Phe-Abu-DAMH-Ile-Phe-NH$_2$

BOC-Phe-His-DAPP-Ile-Phe-OMe

Fp °C

BOC-Ada-His-DAMH-Ile-N-(2-phenylethylamid)

BOC-Phe-Nle-DACP-Ile-DAMH-OMe

| | |
|---|---|
| BOC-Leu-Ala-AHPP-Ile-Gly-OMe | 186-7 |
| BOC-Leu-Ala-AHPP-Ile-Ser-OMe | 192-3 |
| BOC-Leu-Asn-AHPP-Ile-Ser-OMe | 220 |
| BOC-Phe-Asn-AHPP-Gln-Val-OMe | 219 |
| BOC-Gln-Asn-AHPP-Ile-Val-OMe | 182 |
| BOC-Phe-ßAla-AHPP-Ile-Val-OH | |
| BOC-Phe-ßAla-AHPP-Ile-AMPA | |
| CBZ-Phe-Gly -AHPP-Ile-Val-OEt | |
| ETOC-Phe-ßAla-AHCP-Ile-AMPA | |
| BOC-Phe-His -AHMO-Ile-Phe-OMe | 197-8 |
| BOC-Leu-Asn -AHMO-Ile-Ser-OMe | 239-41 |
| BOC-Phe-Asn -AHMO-Gln-Val-OMe | |
| BOC-Gln-Asn -AHMO-Ile-Val-OH | |
| BOC-Phe-ßAla-AHMO-Ile-Ser-OMe | 208-9 |
| BOC-Phe-ßAla-AHMO-Ile-Val-OMe | 211-2 |
| BOC-Phe-ßAla-AHMO-Ile-AMPA | 205-6 |
| BOC-Phe-ßAla-AHMO-Ile-N-(3-pyridylmethyl-amid) | 194-5 |
| BOC-Phe-ßAla-AHMO-Ile-N-benzylamide | 204-5 |
| BOC-Phe-ßAla-AHMO-Ile-NH$_2$ | |
| (3-Thia-4-BOC-Phe-butyryl)-AHMO-Ile-AMPA | 173-4 |
| (3-BOC-Phe-propionyl)-AHMO-Ile-AMPA | |
| BOC-Phe-ßAla-AHMO-Gln-Val-OMe | 219-20 |
| BOC-Phe-Asn-AHMO-Ile-AMPA | |
| BOC-Leu-Asn-AHMO-Ile-AMPA | |
| BOC-Leu-Abu-AHMO-Ile-AMPA | |
| Acetyl-Phe-ßAla-AHMO-Ile-ADPA | |
| BOC-Leu-Ala-AHMO-Ala-Leu-NH$_2$ | |
| Acetyl-Phe-Val-AHMO-Val-Phe-NH$_2$ | |
| BOC-Phe-Asn-AHMO-Asn-Phe-NH$_2$ | |
| (4-BOC-amino-1-piperidinocarbonyl)-Leu-ßAla-AHMO-Ile-AMPA | 170-1 |
| Morpholinocarbonyl-Phe-ßAla-AHMO-Ile-AMPA | |
| Acetyl-Phe-ßAla-AHPH-Ile-AMPA | 209-210 |
| Acetyl-Leu-ßAla-AHPH-Ile-AMPA | |
| BOC-Phe-ßAla-AHPH-Ile-AMPA | 213-5 |
| BOC-Leu-Val -AHPH-Ile-AMPA | 227-8 |
| BOC-Phe-Asn -AHPH-Gln-Val-OMe | 206-8 |
| BOC-Leu-Asn -AHPH-Ile-Gly-OMe | 223-4 |
| BOC-Leu-Val -AHPH-Ile-Val-OMe | 198-9 |
| BOC-ßAla-AHPH-Ile-AMPA | 213-4 |
| BOC-Phe-Asn -AHPH-Ile-AMPA | 232-3 |
| BOC-Leu-Abu -AHPH-Ile-AMPA | 223-4 |
| BOC-Leu-Asn -AHPH-Ile-AMPA | 236-8 |
| BOC-Gln-Asn -AHPH-Ile-AMPA | 216-8 |
| BOC-Phe-ßAla-AHPH-Ile-Val-NH$_2$ | |
| BOC-AHPH-AHPH-Ile-AMPA | 205-6 |
| AHPH-AHPH-Ile-AMPA | 183-4 |
| BOC-Phe-Nle-DACP-Sta-OMe | |

6

## 2) Suppositorien

Man schmilzt ein Gemisch von 500g 3S-Amino-4S-(BOC-Phe-His)-amino-pentanoyl-Ile-Phe-OMe mit 100 g Sojalecithin und 1400 g Kakaobutter, gießt in Formen und läßt erkalten. Jedes Suppositorium enthält 500 mg Wirkstoff.

Analog werden Suppositorien mit folgenden Wirkstoffen hergestellt:

| | Fp °C |
|---|---|
| Morpholinocarbonyl-Leu-βAla-AHPH-Ile-AMPA | |
| Morpholinocarbonyl-Phe-βAla-AHPH-Ile-AMPA | |
| Morpholinocarbonyl-Phe-βAla-AHPH-Ile-Val-NH$_2$ | |
| Morpholinocarbonyl-Phe-βAla-AHCH-Ile-Val-OMe | |
| BOC-Phe-Gly-AHIM-Ile-AMPA | 206 |
| BOC-Phe-βAla-AHIM-Ile-AMPA | 237 |
| (3-Thia-4-BOC-Phe-butyryl)-AHIM-Ile-AMPA | 196 |
| (3-BOC-Phe-propionyl)-AHIM-Ile-AMPA | 202 |
| (N-Benzyl-N-isopentyl-carbamoyl)-Gly-AHIM-Ile-3-pyridylmethyl-amid | 172 |
| (N-Benzyl-N-isopentyl-carbamoyl)-Gly-AHIM-Ile-AMPA | 190-1 |
| (N-Benzyl-N-isopentyl-carbamoyl)-βAla-AHIM-Ile-AMPA | 99 |
| BOC-Phe-His-AHIM-Leu-5-aminomethyltetrazol | 193 |
| (4-BOC-amino-1-piperidinocarbonyl)-Leu-βAla-AHIM-Ile-AMPA | 219-20 |
| BOC-Phe-βAla-AHIM-Ile-Ser-OMe | 205-6 |
| BOC-Phe-βAla-AHIM-Ile-Val-OMe | 228-9 |
| BOC-Phe-D-Ala-AHIM-Ile-AMPA | 217-8 |
| BOC-Phe-Ala-AHIM-Ile-AMPA | 255-6 |
| BOC-Gln-Asn-AHIM-Ile-AMPA | 214-5 |
| (4-Amino-1-piperidinocarbonyl)-Leu-βAla-AHIM-Ile-AMPA | 103-5 |
| (4-BOC-amino-1-piperidinocarbonyl)-Phe-βAla-AHIM-Ile-AMPA | 199-200 |
| (4-Amino-1-piperidinocarbonyl)-Phe-βAla-AHIM-Ile-AMPA | 182-3 |
| Morpholinocarbonyl-Phe-βAla-AHIM-Ile-AMPA | |
| Morpholinocarbonyl-Leu-βAla-AHIM-Ile-AMPA | |
| Morpholinocarbonyl-Leu-βAla-AHIM-Leu-AMPA | |
| 4S-hydroxy-5S-(BOC-Phe-His)-amino-7-methyloctanoyl-Ile-Phe-OMe | |
| 2-Benzyl-4-phenyl-butyryl-Ala-AHCP-Ile-DAMH-OMe | |
| 3S-hydroxy-4S-(BOC-Phe-His)-aminooctansäure | |
| CBZ-His-Sta-Ile-Phe-OH | |

## 3) Tabletten

a. Ein Gemisch von 1 kg CBZ-Leu-Sta-Leu-Phe-OH, 4 kg Lactose, 1,2 kg Maisstärke, 200 g Talk und 100 g Magnesiumstearat wird in üblicher Weise zu Tabletten verpreßt, derart daß jede Tablette 200 mg Wirkstoff enthält.

Analog werden Tabletten hergestellt mit folgenden Wirkstoffen:

Fp °C

BOC-Leu-ßAla-AHIM-ßAla-Leu-NH$_2$
BOC-Leu-ßAla-AHIM-Ala-AMPA
Acetyl-Phe-ßAla-AHIM-ßAla-Phe-NH$_2$
Morpholinocarbonyl-Leu-ßAla-AHIM-ßAla-Val-OMe
BOC-Leu-ßAla-AHIM-Gly-AMPA
Acetyl-Leu-ßAla-AHIM-Gly-AMPA
CBZ-ßAla-AHIM-Ile-AMPA
BOC-Leu-ßAla-AHIM-Ile-NH$_2$
BOC-Leu-ßAla-AHIM-Ile-N-i.Butylamid
Leu-ßAla-AHIM-Ile-AMPA
CBZ-ßAla-AHIM-Ile-N-(2-phenethylamid )
Acetyl-Leu-ßAla-AHIM-Leu-N-(3-pyridylmethyl-amid )
BOC-Phe-Asn-AHIM-Ile-AMPA                        256-7
BOC-Leu-Asn-AHIM-Ile-AMPA                        261-2
BOC-Phe-Abu-AHIM-Ile-AMPA                        260-3
(2-Benzyl-4-oxo-5,5-dimethylhexanoyl)-ßAla-
AHIM-Ile-AMPA                                     145
IPOC-Leu-ßAla-AHIM-Ile-AMPA
ETOC-Leu-ßAla-AHIM-Ile-Val-NH$_2$
IPOC-Leu-ßAla-AHIM-Ile-N-(3-pyridylmethyl-amid )
BOC-Mal-ßAla-AHIM-Ile-AMPA
Morpholinocarbonyl-Leu-ßAla-AHIM-Ile-Val-OMe
(4-Amino-1-piperidinocarbonyl)-Leu-ßAla-
AHIM-Ile-Val-NH$_2$
(4-Amino-1-piperidinocarbonyl)-Leu-ßAla-
AHIM-Ala-Val-NH$_2$
(4-Amino-1-piperidinocarbonyl)-Leu-ßAla-
AHIM-Val-Val-NH$_2$
(4-Amino-1-piperidinocarbonyl)-Leu-ßAla-
AHIM-ßAla-Val-NH$_2$
(4-Amino-1-piperidinocarbonyl)-Leu-ßAla-
AHIM-Leu-Val-NH$_2$
Morpholinocarbonyl-Leu-Asn-AHIM-Ile-Val-OMe
BOC-AHPH-AHIM-Ile-AMPA

BOC-Phe-ßAla-AHIP-Ile-AMPA
BOC-Leu-ßAla-AHIP-Ile-Val-NH₂
(4-Amino-1-piperidinocarbonyl)-Phe-ßAla-AHIP-Ile-AMPA
(4-Amino-1-piperidinocarbonyl)-Leu-ßAla-AHIP-Ile-AMPA
(4-Amino-1-piperidinocarbonyl)-Leu-ßAla-AHIP-Leu-AMPA
Morpholinocarbonyl-Leu-ßAla-AHIP-Leu-Val-OMe
(4-Amino-1-piperidinocarbonyl)-Leu-ßAla-AHIP-Leu-Val-NH₂
(4-Amino-1-piperidinocarbonyl)-Leu-ßAla-AHIP-Ala-Val-NH₂
(4-Amino-1-piperidinocarbonyl)-Leu-ßAla-AHIP-Val-Val-NH₂
(4-Amino-1-piperidinocarbonyl)-Leu-ßAla-AHIP-ßAla-Val-NH₂
3-Phenylpropionyl-His-Sta-Leu-p-chlorbenzyl-amid
BOC-Phe-His-DACP-Ile-N-(2-phenylethylamid)
BOC-Phe-Gly-AHCP-Ile-N-(3-pyridyl-methyl-amid)

4) Dragees

Analog Beisiel 3 werden Tabletten gepreßt, die anschließend in üblicher Weise mit einem Überzug aus Saccharose, Maisstärke, Talk, Tragant und Farbstoff überzogen werden.

5) Kapseln

500 g BOC-Phe-His-AHCP-Leu-OH werden in üblicher Weise in Hartgelatinekapseln gefüllt, so daß jede Kapsel 500 mg Wirkstoff enthält.

**Ansprüche**

1) Verwendung von Aminosäurederivaten, die die Aktivität von Retroviren-Proteasen hemmen und das Strukturelement der Formel I
-NR¹-CHR²-CR³-(CHR⁴)ₙ-CO-     I
aufweisen, worin
$R^1$, $R^4$, $R^5$, $R^6$ jeweils H oder Alkyl,
$R^2$ H, Alkyl, jeweils substituiertes oder unsubstituiertes Aryl, Arylalkyl, Cycloalkyl, Cycloalkylalkyl, Het oder Het-alkyl,
$R^3$ (H, OH), (H, NR⁵R⁶) oder O,
Het einen 5- oder 6-gliedrigen N-, S-oder O-haltigen gesättigten oder ungesättigten Heterocyclus und
n 1 oder 2
bedeuten, oder von deren physiologisch unbedenklichen Salzen zur Herstellung eines Arzneimittels für die Behandlung von durch Retroviren verursachte Erkrankungen.

2) Verwendung von Aminosäurederivaten, die die Aktivität von Retroviren-Proteasen hemmen und das Strukturelement der Formel I aufweisen oder von deren physiologisch undedenklichen Salzen bei der Bekämpfung von durch Retroviren verursachte Erkrankungen.

3) Verwendung von Aminosäurederivaten nach Anspruch 1 zur Herstellung eines Arzneimittels für die Behandlung von AIDS.

9

4) Verwendung von Aminosäurederivaten nach Anspruch 2 bei der Bekämpfung von AIDS.

## EUROPÄISCHER TEILRECHERCHENBERICHT,

Europäisches Patentamt

der nach Regel 45 des Europäischen Patent-übereinkommens für das weitere Verfahren als europäischer Recherchenbericht gilt

Nummer der Anmeldung

EP 89 11 7881

## EINSCHLÄGIGE DOKUMENTE

| Kategorie | Kennzeichnung des Dokuments mit Angabe; soweit erforderlich, der maßgeblichen Teile | Betrifft Anspruch | KLASSIFIKATION DER ANMELDUNG (Int. Cl.4) |
|---|---|---|---|
| | Keine sinnvolle Recherche möglich EPÜ Regel 45; in Verbindung mit EPÜ Art. 83,84; EPÜ Regel 27(1) f. | | A 61 K 37/64 A 61 K 37/02 |

RECHERCHIERTE SACHGEBIETE (Int. Cl.4)

A 61 K 37

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Annexe B

| Recherchenort | Abschlußdatum der Recherche | Prüfer |
|---|---|---|
| Den Haag | 21-12-1989 | ISERT |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Unvollständige Recherche

Grund für die Beschränkung der Recherche:

Die beanspruchte therapeutische Wirkung ist nicht
gestützt durch pharmakologische Beispiele, siehe
EPÜ-Art. 83,84; EPÜ-Regel 27 (1) f).
Die beanspruchten Verbindungen (vgl. Anspruch 1) sind
unzureichend definiert: Die Angabe ihrer biochemischen
Aktivität und der Besitz eines bestimmten Strukturelements ist für eine exakte chemische Charakterisierung
nicht genügend; die Bedeutung des Substituenten "R2"
ist undeutlich, siehe EPÜ-Art. 83,84.